(19) Europäisches Patentamt
European Patent Office
Office européen des brevets

(11) Veröffentlichungsnummer: **0 403 884 A2**

(12) **EUROPÄISCHE PATENTANMELDUNG**

(21) Anmeldenummer: **90110763.1**

(22) Anmeldetag: **07.06.90**

(51) Int. Cl.[5]: **C07C 255/36, A01N 37/34, C07C 255/31, C07C 255/12**

(30) Priorität: **20.06.89 DE 3920027**

(43) Veröffentlichungstag der Anmeldung:
**27.12.90 Patentblatt 90/52**

(84) Benannte Vertragsstaaten:
**BE DE DK FR GB IT NL**


(71) Anmelder: **BAYER AG**

**D-5090 Leverkusen 1 Bayerwerk(DE)**

(72) Erfinder: **Schade, Gerold, Dr.**
**Hahnenweg 8**
**D-5000 Köln 80(DE)**
Erfinder: **Wachtler, Peter, Dr.**
**Morgengraben 4**
**D-5000 Köln 80(DE)**
Erfinder: **Frie, Monika, Dr.**
**Im Alten Driesch 1**
**D-5068 Odenthal-Osenau(DE)**
Erfinder: **Paulus, Wilfried, Dr.**
**Deswatinesstrasse 90**
**D-4150 Krefeld 1(DE)**
Erfinder: **Dutzmann, Stefan, Dr.**
**Leinenwebereg 33**
**D-4000 Düsseldorf 14(DE)**


(54) **Dibrompropanol-Derivate, Verfahren zur Herstellung und ihre Verwendung.**


(57) Die Erfindung betrifft neue Dibrompropanol-Derivate der Formel

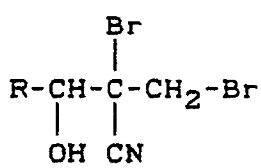

(I),

in der
R für einen gegebenenfalls substituierten Alkyl-, Cycloalkyl-, Aryl- oder Heteroaryl-Rest steht,
ein Verfahren zu ihrer Herstellung und ihre Verwendung als Mikrobizide für den Materialschutz.


EP 0 403 884 A2

## Dibrompropanol-Derivate, Verfahren zur Herstellung und ihre Verwendung

Die Erfindung betrifft neue 2-Cyano-2,3-dibrom-propanol-Derivate, ein Verfahren zu ihrer Herstellung und ihre Verwendung als Mikrobizide für den Materialschutz.

Es ist bereits bekannt, bromierte Propanolderivate, nämlich 2-Brom-2-nitro-3-hydroxy-propanol, als Konservierungsstoff für funktionelle Flüssigkeiten zu verwenden (siehe Seifen-Öle-Fette-Wachse 114 (1988) S. 319-323). Die Wirkung dieses Mikrobizides gegenüber Schimmelpilzen ist jedoch unbefriedigend; es wird daher empfohlen, diese Verbindung nur in Kombination mit sehr gut fungizid wirksamen Verbindungen wie Isothiazolinonen zur Konservierung funktioneller Flüssigkeiten anzuwenden (siehe Seifen-Öle-Fette-Wachse loc.cit. S. 321-322). Ein weiterer Nachteil des 2-Brom-2-nitro-3-hydroxy-propanols bei seiner Verwendung als Konservierungsstoff ist ferner, daß es bei pH-Werten >7,5 unter Abspaltung von Formaldehyd und Nitrit, d.h. ökologisch bedenklichen Verbindungen, hydrolysiert.

Überraschenderweise wurde nun gefunden, daß bestimmte neue Dibrompropanol-Derivate eine wesentlich verbesserte mikrobizide Wirksamkeit aufweisen und außerdem bei ihrer Hydrolyse keine toxischen Verbindungen bilden. Außerdem wurde gefunden, daß einige dieser neuen Dibrompropanol-Derivate eine mit bekannten Fungiziden noch nicht erreichte Wirkung gegen Fusarien, z.B. Fusarium nivale, und einige eine gute Wirkung gegen Mehltau aufweisen.

Die Erfindung betrifft daher Dibrompropanol-Derivate der Formel

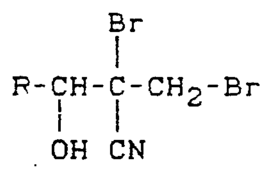

(I),

in der

R für einen gegebenenfalls substituierten Alkyl-, Cycloalkyl-, Aryl- oder Heteroaryl-Rest steht.

Für R seien beispielsweise genannt:

als gegebenenfalls substituierte Alkylreste: $C_1$-$C_{12}$-Alkylreste wie der Methyl-, Ethyl-, n-Propyl-, i-Propyl-, n-Butyl-, tert.-Butyl-, n-Hexyl-, i-Octyl- und der n-Dodecyl-Rest; durch Halogenatome wie Fluor, Chlor oder Brom, $C_1$-$C_4$-Alkoxy-, wie Methoxy oder Ethoxy; $C_1$-$C_4$-Alkylmercapto wie Methylmercapto, Trifluormethylmercapto und Dichlorfluormethylmercapto-Reste, substituierte $C_1$-$C_4$-Alkylreste wie der 2-Methoxyethyl-, 2-Ethoxyethyl-, 2-Chlorethyl-, 2-Trifluormethylmercaptoethyl- und 2,3-Dibrompropyl-Rest;

als gegebenenfalls substituierte Cycloalkylreste der Cyclohexylrest und durch Halogen, insbesondere Chloratome und/oder $C_1$-$C_4$-Alkylgruppen substituierte Cyclohexylreste wie der 4-Methylcyclohexyl-, 2,6-Dimethylcyclohexyl- und 4-tert.-Butylcyclohexylrest;

als gegebenenfalls substituierte Arylreste insbesondere der Phenylrest und durch Halogen, Nitro oder Cyano substituierte Phenylreste wie der 2-Chlorphenyl-, 4-Chlorphenyl-, 4-Bromphenyl-, 4-Nitrophenyl- und der 2,6-Dichlorphenyl-Rest;

als gegebenenfalls substituierte Heteroarylreste der 2-, 3- oder 4-Pyridylrest und durch Halogen und/oder $C_1$-$C_4$-Alkylgruppen substituierte Pyridylreste wie der 6-Chlorpyridyl-2-Rest.

Bevorzugt steht R für einen $C_1$-$C_6$-Alkyl- oder einen durch Chlor oder Brom substituierten Phenyl-Rest, besonders bevorzugt für einen $C_3$-$C_6$-Alkylrest.

Als Vertreter der erfindungsgemäßen Dibrompropanol-Derivate der Formel (I) seien folgende Verbindungen genannt: 1,2-Dibrom-2-cyano-3-hydroxy-butan, 1,2-Dibrom-2-cyano-3-hydroxy-pentan, 1,2-Dibrom-2-cyano-3-hydroxy-hexan, 1,2-Dibrom-2-cyano-3-hydroxy-4-methyl-pentan, 1,2-Dibrom-2-cyano-3-hydroxy-4,4-dimethyl-pentan, 1,2-Dibrom-2-cyano-3-hydroxy-nonan, 1,2-Dibrom-2-cyano-3-hydroxy-3-phenyl-propan, 1,2-Dibrom-2-cyano-3-hydroxy-3-(4-brom-phenyl)-propan, 1,2-Dibrom-2-cyano-3-hydroxy-3-(2-chlorphenyl)-propan, 1,2-Dibrom-2-cyano-3-hydroxy-3-(4-chlorphenyl)-propan, 1,2-Dibrom-2-cyano-3-hydroxy-3-(4-nitrophenyl)-propan, 1,2-Dibrom-2-cyano-3-hydroxy-3-(2,6-dichlorphenyl)-propan, 1,2-Dibrom-2-cyano-3-hydroxy-3-cyclohexylpropan.

Die erfindungsgemäßen Dibrompropanol-Derivate der Formel (I) werden durch Bromieren von Acrylverbindungen der Formel

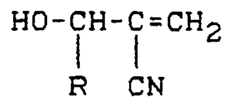 (II),

in der
R die unter Formel (I) angegebene Bedeutung hat, erhalten.

Die Acrylverbindungen der Formel (II) sind bekannt und z.B. in der DE-OS 2 155 113 beschrieben.

Die Erfindung betrifft auch dieses Verfahren zur Herstellung der Dibrompropanol-Derivate der Formel (I); das Verfahren ist dadurch gekennzeichnet, daß man die Acryl derivate der Formel (II), gegebenenfalls in Gegenwart eines unter den Reaktionsbedingungen inerten organischen Lösungsmittels, bromiert.

Als unter den Reaktionsbedingungen inerte organische Lösungsmittel seien beispielsweise aliphatische Chlorkohlenwasserstoffe wie Methylenchlorid, Ethylenchlorid, Chloroform und Tetrachlorkohlenstoff, aromatische Chlorkohlenwasserstoffe wie Chlorbenzol und ortho-Dichlorbenzol oder Eisessig genannt.

Die Bromierung wird bei Temperaturen von 0 bis 80° C, vorzugsweise bei Temperaturen von 20 bis 60° C vorgenommen.

Für die Bromierung wird eine solche Menge Brom verwendet, daß auf 1 Mol zu halogenierende Acrylverbindung der Formel (II) 1 bis 1,2 Mol Brom entfallen.

Die Zugabe des Broms und die Aufarbeitung der Bromierungsmischung erfolgt in aus der präparativen Chemie bekannten Weise.

Die erfindungsgemäßen Dibrompropanole der Formel (I) zeichnen sich durch eine starke mikrobizide Wirkung und eine gute fungizide Wirksamkeit, insbesondere gegen Fusarien und Mehltau aus. Sie sind den bekannten bromierten Propanolderivaten sowohl in Bezug auf ihre fungizide Wirkung als auch in Bezug auf die Breite ihres Wirkungsspektrums wesentlich überlegen. Die Erfindung betrifft daher auch die Verwendung der erfindungsgemäßen Dibrompropanol-Derivate der Formel (I) als Mikrobizide zum Schutz technischer Materialien und als Fungizide im Pflanzenschutz.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), pflanzenschädigende Pilze sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Botrytis, wie Botrytis cinera,
Chaetomium, wie Chaetomium globosum,
Coniophora, wie Coniophora puteana,
Lentinus, wie Lentinus tigrinus,
Penicillium, wie Penicillium glaucum,
Piricularia, wie Piricularia oryzae,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus

Fungizide werden im Pflanzenschutz zur Bekämpfung von Plasmodiophoromycetes, Oomycetes, Chytridiomycetes, Zygomycetes, Ascomycetes, Basidiomycetes, Deuteromycetes eingesetzt.

Als Erreger von pilzlichen Erkrankungen, die unter die oben aufgezählten Oberbegriffe fallen, seien beispielsweise genannt:

Pythium-Arten, wie beispielsweise Pythium ultimum; Phytophthora-Arten, wie beispielsweise Phytophthora infestans; Pseudoperonospora-Arten, wie beispielsweise Pseudoperonospora humuli oder Pseudoperonospora cubensis;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis; Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea

(Konidienform: Drechslera, Syn: Helminthosporium);

Cochliobolus-Arten, wie beispielsweise Cochliobolus sativus

(Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielsweise Pseudocercosporella herpotrichoides.

Besonders bewährt haben sich einige der erfindungsgemäßen Wirkstoffe zur Bekämpfung von Fusarium-Arten, wie beispielsweise Fusarium nivale, inbesondere an Getreide. Weiterhin zeigen einige der erfindungsgemäßen Wirkstoffe gute fungizide Wirkungen gegen echten Mehltau z.B. an Reben (Uncinula), Äpfeln und Getreide, wie Gerste und Weizen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Je nach Anwendungsgebiet können die erfindungsgemäßen Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen angewendet werden Beispielsweise seien die folgenden Wirkstoffe genannt: Benzylalkoholmono(poly)-hemiformal und andere Formaldehyd abspaltende Verbindungen, Isothiazolinon-Derivate, Benzisothiazolinon, p-Hydroxybenzoesäureester, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, 2-Thiocyanatomethylthio-benzthiazol, Phenolderivate, wie

2-Phenylphenol, (2,2'-Dihydroxy-5,5'-dichlor)diphenylmethan und 3-Methyl-4-chlor-phenol, N-Trihalogenmethylthio-Verbindungen, wie Folpet, Fluorfolpet und Dichlorfluamid, Azolfungizide wie Triadimefon, Triadimenol und Bitertanol.

## Beispiel 1

Die Lösung von 63,8 g (0,5 Mol) 2-(1-Hydroxybutyl)-acrylnitril in 200 ml Chloroform wird bei 60° C innerhalb von 1,5 Stunden unter Rühren tropfenweise mit 79,9 g (0,5 Mol) Brom versetzt. Nach beendeter Zugabe wird die Reaktionslösung 3 Stunden bei 60° C gerührt. Anschließend wird die Lösung auf Raumtemperatur abgekühlt und dann 3 x mit je 20 ml 10 %iger Natriumbisulfitlösung gewaschen. Nach dem Trocknen der Lösung über Natriumsulfat wird die Lösung am Rotationsverdampfer eingeengt.

Es werden 133,7 g (93,8 % der Theorie) eines Öles erhalten, das gemäß gaschromatographischer Analyse zu 84,1 % aus 1,2-Dibrom-2-cyano-3-hydroxyhexan besteht. Die Verbindung wird durch ihr Massenspektrum: $M^+$ 285 charakterisiert.

## Beispiel 2

Die Lösung von 31,7 g (0,2 Mol) 2-(α-Hydroxybenzyl)-acrylnitril in 150 ml Chloroform wird bei 60° C innerhalb von 1 Stunde unter Rühren mit der Lösung von 32 g (0,2 Mol) Brom in 50 ml Chloroform tropfenweise versetzt. Nach beendeter Zugabe wird die Reaktionsmischung $3^1/_2$ Stunden bei 60° C gerührt. Anschließend wird sie auf Raumtemperatur abgekühlt, mit 20 ml Natriumbisulfitlösung verrührt, die wäßrige Phase abgetrennt und die organische Phase über Natriumsulfat getrocknet. Das nach dem Entfernen des Lösungsmittels erhaltene Rohprodukt wird säulenchromatographisch gereinigt (Laufmittel: Toluol/Essigester 9:1; Kieselgel).

Es werden 31,7 g (= 49,7 % der Theorie) 1,2-Dibrom-2-cyano-3-hydroxy-3-phenyl-propan in Form eines gelben Öles erhalten.

Die Verbindung wird durch ihr IR-Spektrum charakterisiert (3470 $cm^{-1}$ -OH; 2255 $cm^{-1}$ -CN).

In der nachstehenden Tabelle sind die physikalischen Kenndaten der Dibrompropanole der Formel (I) zusammengestellt, die in analoger Weise erhalten wurden und in denen R die ebenfalls in der Tabelle angegebene Bedeutung hat.

Tabelle 1

| Beispiel | R | Physikalische Kenngrößen |
|---|---|---|
| 3 | $CH_3$- | $^1$H-NMR: $\delta$ = 1,51 ppm -$CH_3$<br>$\delta$ = 2,27 ppm -OH |
| 4 | $C_2H_5$- | $^1$H-NMR: $\delta$ = 1,15 ppm -$CH_3$<br>$\delta$ = 2,26 ppm -OH<br>$\delta$ = 3,7 - 41 ppm <u>H</u>C-OH, $CH_2$-Br |
| 5 | 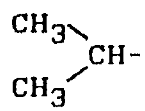 | $^1$H-NMR: $\delta$ = 1,15 ppm -$CH_3$ |
| 6 | n-$C_6H_{13}$- | IR: 3470 $cm^{-1}$ -OH<br>2240 $cm^{-1}$ -CN |
| 7 | t-$C_4H_9$ | IR: 3480 $cm^{-1}$ -OH<br>2240 $cm^{-1}$ -CN |
| 8 | 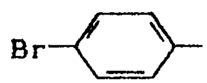 | Fp.: 119° C |
| 9 | 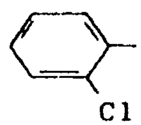 | Fp.: 123-128° C |
| 10 | 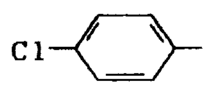 | IR: 3470 $cm^{-1}$ -OH<br>2250 $cm^{-1}$ -CN<br>1600 $cm^{-1}$ arom. =C-H |

**Tabelle 1 (Fortsetzung)**

| Beispiel | R | Physikalische Kenngrößen |
|---|---|---|
| 11 | 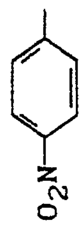 | Fp.: 124-26° C |
| 12 | 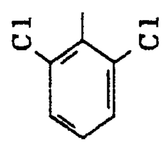 | Massenspektrum: m/e = 228($M^+$-$Br_2$) |
| 13 | 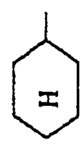 | IR: 3470 $cm^{-1}$ -OH<br>2230 $cm^{-1}$ -CN |

## Anwendungsbeispiele

### Beispiel 14

Zum Nachweis der Wirksamkeit gegen Pilze wurden die minimalen Hemm-Konzentrationen (MHK) erfindungsgemäßer Dibrompropanole bestimmt:

Ein Agar, der aus Bierwürze und Pepton hergestellt wird, wird mit erfindungsgemäßen Wirkstoffen in Konzentrationen von 0,1 mg/l bis 5.000 mg/l versetzt. Nach Erstarren des Agars erfolgt Kontamination mit Reinkulturen der in der Tabelle aufgeführten Testorganismen. Nach 2-wöchiger Lagerung bei 28° C und 60

bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle 2 angegeben.

Tabelle 2

| MHK's [mg/l] bei der Einwirkung verschiedener Brompropanole auf Pilze | | | | | | | | | | | |
|---|---|---|---|---|---|---|---|---|---|---|---|
| | erfindungsgemäße Verbindungen gemäß Beispiel | | | | | | | | | | |
| Testorganismen | 1 | 2 | 3 | 4 | 5 | 6 | 7 | 10 | 12 | 13 | Vergleich* |
| Alternaria tenuis | 35 | <1 | 50 | 20 | 20 | 150 | 20 | 75 | 35 | 75 | 200 |
| Aspergillus niger | 200 | <20 | 200 | 200 | 200 | 100 | 200 | 150 | 200 | 100 | >1000 |
| Aureobasidium pullulans | 75 | 20 | 100 | 50 | 50 | 75 | 150 | 10 | 50 | 50 | 750 |
| Chaetomium globosum | 200 | 200 | 200 | 200 | 200 | 100 | 200 | 200 | 150 | 50 | 750 |
| Cladosporium cladosporioides | 20 | <1 | 50 | 15 | 20 | 20 | 20 | 20 | 20 | 7,5 | 1000 |
| Lentinus tigrinus | 50 | 2 | 100 | 50 | 50 | 20 | 35 | 200 | 20 | 20 | 750 |
| Penicillium glaucum | 50 | <20 | 200 | 100 | 50 | 20 | 50 | 100 | 50 | <20 | 1000 |
| Sclerophoma pityophila | 20 | 7,5 | 20 | 20 | 20 | 35 | 10 | 2 | 20 | 20 | 500 |
| Trichoderma viride | 200 | 200 | 350 | 200 | 200 | 200 | 500 | 200 | 200 | 500 | >1000 |

* 2-Brom-2-nitro-3-hydroxy-propanol

Beispiel 15

Wirkung gegen Bakterien

Ein Agar, der als Nährmedium Bouillon enthält, wird mit erfindungsgemäßen Wirkstoffen in Konzentrationen von 1 bis 5.000 ppm versetzt. Darauf infiziert man das Nährmedium jeweils mit den in Tabelle II aufgeführten Testorganismen und hält das infizierte Medium 2 Wochen bei 28° C und 60 bis 70 % rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle 3 wiedergegeben.

Tabelle 3

| Angabe der MHK-Werte in mg/l bei der Einwirkung der unten angegebenen Wirkstoffe auf Bakterien. | | | | | | |
|---|---|---|---|---|---|---|
| | MHK in mg/l der Wirkstoffe | | | | | |
| Testorganismen | Verbindung des Beispiels Nr. | | | | | |
| | 1 | 2 | 3 | 4 | 5 | 6 |
| Escherichia coli | 200 | 1000 | 200 | 150 | 750 | 500 |
| Staphylococcus aureus | 200 | 1000 | 200 | 500 | 500 | 100 |

Beispiel 16 (Wirkung gegen Schleimorganismen)

Die zu testenden Verbindungen werden, in wenig Aceton gelöst, in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, zur Anwendung gebracht. Zu Beginn des Testes werden die einzelnen Nährlösungen mit Schleimorganismen (ca. $10^6$ Keime/ml), die aus bei der

Polyamid-Herstellung verwendeten Spinnwasser-Kreisläufen isoliert wurden, infiziert. Nährlösungen, die den zu testenden lodpropargylether in mindestens minimaler Hemmkonzentration (MHK) enthielten, sind nach 3-wöchiger Kultur bei Raumtemperatur noch völlig klar; die in wirkstofffreien Nährlösungen nach 3 bis 4 Tagen eintretende durch die starke Vermehrung der Mikroben hervorgerufene Schleimbildung und die damit verbundene Trübung wird in ihnen unterbunden.

Tabelle 4

| MHK-Wert [mg/l] der erfindungsgemäßen Dibrompropanole bei der Einwirkung auf Schleimorganismen. | |
|---|---|
| Dibrompropanol gemäß Beispiel | MHK [mg/l] |
| 1 | 5 - <10 |
| 2 | 5 - <10 |
| 3 | 5 |
| 4 | >5 - <10 |

Beispiel 17 (Wirkung gegen Algen)

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaeodactylum tricornutum Bohlin) wird unter Durchperlen von Luft in Allens Nährlösung (Arch. Mikrobiol. 17, 34 bis 53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemäßer Wirkstoffe erkennt man an dem Entfärben der Nährlösung.

Tabelle 5

| Algen-abtötende Konzentrationen [mg/l] der erfindungsgemäßen Dibrompropanole | |
|---|---|
| Verbindung des Beispiels Nr. | abtötende Konzentrationen [mg/l] |
| 1 | 50 - <100 |
| 2 | >50 - <100 |
| 3 | 50 |
| 4 | 50 - <100 |
| Vergleich: | |
| 2-Brom-2-nitro-3-hydroxy-propanol | >100 |

Beispiel 18

Fusarium nivale-Test (Roggen) / Saatgutbehandlung

Die Anwendung der Wirkstoffe erfolgt als Trockenbeizmittel. Diese werden zübereitet durch Vermischen (Strecken) des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Roggen sät man mit 2 x 100 Korn 1 cm tief in eine Standarderde und kultiviert ihn im Gewächshaus bei einer Temperatur von ca. 10° C und einer relativen Luftfeuchtigkeit von ca. 95 % in Saatkästen, die täglich 15 Stunden dem Licht ausgesetzt werden.

Ca. 3 Wochen nach der Aussaat erfolgt die Auswertung der Pflanzen auf Symptome des Schneeschimmels.

Eine deutliche Überlegenheit in der Wirksamkeit gegenüber dem Stand der Technik zeigen bei diesem Test z.B. die Verbindungen gemäß folgender Herstellungsbeispiele:

Tabelle

Fusarium nivale-Test (Roggen) / Saatgutbehandlung

| Wirkstoff | Wirkstoffaufwandmenge in mg/kg Saatgut | Wirkungsgrad in % der unbehandelten Kontrolle |
|---|---|---|
| 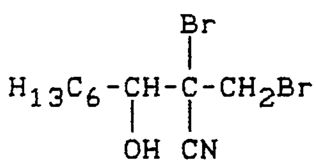 (Beispiel 6) | 500 | 89 |
| 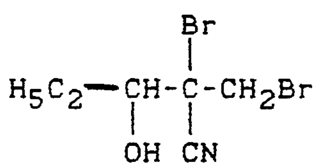 (Beispiel 4) | 500 | 91 |
| gemäß Stand der Technik: (US-PS 4 446 076) | | |
| 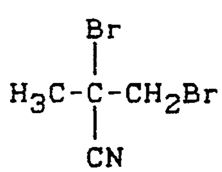 | 500 | 66 |

Beispiel 19

Erysiphe-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20° C und einer relativen Luftfeuchtigkeit von ca. 80 % aufgetsellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele:

Tabelle

Erysiphe-Test (Gerste) / protektiv

| Wirkstoff | Wirkstoffkonzentration in der Spritzbrühe in Gew.-% | Wirkungsgrad in % der unbehandelten Kontrolle |
|---|---|---|
| 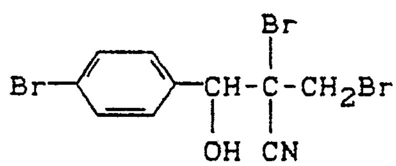 (Beispiel 8) | 0,00025 | 75 |
| gemäß Stand der Technik: (US-PS 4 446 076) | | |
| 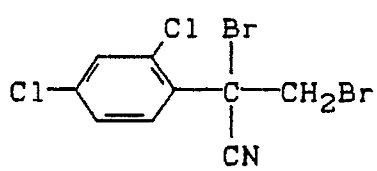 | 0,00025 | 0 |

Beispiel 20

Erysiphe-Test (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit

den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20° C und einer relativen Luftfeuchtigkeit von ca. 80 % aufgetsellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele:

<u>Tabelle</u>

Erysiphe-Test (Weizen) / protektiv

| Wirkstoff | Wirkstoffkonzentration in der Spritzbrühe in Gew.-% | Wirkungsgrad in % der unbehandelten Kontrolle |
|---|---|---|
| 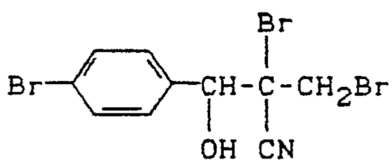 (Beispiel 8) | 0,0025 | 75 |
| gemäß Stand der Technik: (US-PS 4 446 076) 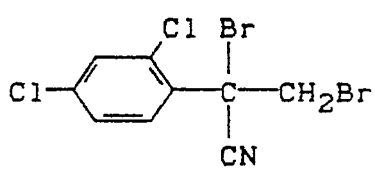 | 0,0025 | 16 |

**Ansprüche**

1. Dibrompropanol-Derivate der Formel

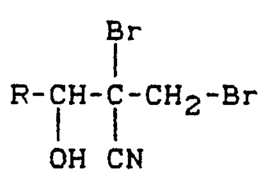 (I),

in der

R für einen gegebenenfalls substituierten Alkyl-, Cycloalkyl-, Aryl- oder Heteroaryl-Rest steht.

2. Dibrompropanol-Derivate gemäß Anspruch 1 dadurch gekennzeichnet, daß R für einen $C_1$-$C_6$-Alkylrest steht.

3. Dibrompropanol-Derivate gemäß Anspruch 1 dadurch gekennzeichnet, daß R für einen $C_3$-$C_6$-Alkylrest steht

4. Verfahren zur Herstellung von DibrompropanolDerivaten der Formel

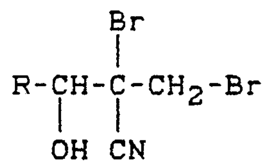

(I),

in der
R für einen gegebenenfalls substituierten Alkyl-, Cycloalkyl-, Aryl- oder Heteroaryl-Rest steht,
dadurch gekennzeichnet, daß man Acrylverbindungen der Formel

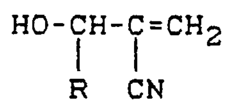

(II),

in der
R die unter Formel (I) angegebene Bedeutung hat,
gegebenenfalls in Gegenwart eines unter den Reaktionsbedingungen inerten organischen Lösungsmittels, in an sich bekannte Weise bromiert.

5. Verwendung der Dibrompropanol-Derivate gemäß Ansprüchen 1, 2 oder 3 als Mikrobizide im Materialschutz.

6. Verwendung der Dibrompropanol-Derivate gemäß Ansprüchen 1, 2 oder 3 als Fungizide im Pflanzenschutz.